**Europäisches Patentamt**

**European Patent Office**

**Office européen des brevets**

(19)

(11) Veröffentlichungsnummer: **0 036 623**
**B1**

(12) **EUROPÄISCHE PATENTSCHRIFT**

(45) Veröffentlichungstag der Patentschrift:
**21.09.83**

(21) Anmeldenummer: **81101996.7**

(22) Anmeldetag: **18.03.81**

(51) Int. Cl.³: **B 01 J 37/00**, B 01 J 27/18, C 07 D 307/60

(54) **Verfahren zur Herstellung von Katalysatoren für die Gasphaseoxidation von gesättigten und/oder ungesättigten C4-Kohlenwasserstoffen zu Maleinsäureanhydrid.**

(30) Priorität: **20.03.80 DE 3010710**

(43) Veröffentlichungstag der Anmeldung:
**30.09.81 Patentblatt 81/39**

(45) Bekanntmachung des Hinweises auf die Patenterteilung:
**21.09.83 Patentblatt 83/38**

(84) Benannte Vertragsstaaten:
**BE CH DE FR GB IT LI LU NL SE**

(56) Entgegenhaltungen:
**FR-A-2189493**
**FR-A-2351702**
**US-A-3538122**
**US-A-4016105**

(73) Patentinhaber: **CHEMISCHE WERKE HÜLS AG, Postfach 1320, D-4370 Marl 1 (DE)**

(72) Erfinder: **Neubold, Kurt, Dr., Lindenstrasse 57, D-4390 Gladbeck (DE)**
Erfinder: **Gollmer, Klaus-Dieter, Dr., Brahmweg 20, D-4250 Bottrop-Kirchhellen (DE)**

(74) Vertreter: **Steil, Hanna, Dipl.-Chem., RSP PATENTE - PB 15 Postfach 1320, D-4370 Marl 1 (DE)**

## Verfahren zur Herstellung von Katalysatoren für die Gasphaseoxidation von gesättigten und/oder ungesättigten C₄-Kohlenwasserstoffen zu Maleinsäureanhydrid

Bei der Herstellung von Maleinsäureanhydrid hat aus wirtschaftlichen und ökologischen Gründen der Ersatz von Benzol durch C₄-Kohlenwasserstoffe als Ausgangsmaterial grosse Beachtung gefunden. Es sind daher zahlreiche Verfahren zur Gasphaseoxidation aus C₄-Kohlenwasserstoffen bekannt geworden. Da die C₄-Kohlenwasserstoffe nach der Leichtigkeit, mit der sie oxidierbar sind, in die drei Gruppen Butadien, Butene und Butan eingeteilt werden können, erfordert die Oxidation dieser Gruppen in der Regel auch unterschiedliche Katalysatoren und Reaktionsbedingungen zur Durchführung dieser Reaktion. Die Reaktionsfreudigkeit nimmt in der oben angegebenen Reihenfolge vom Butadien über die Butene zum Butan ab. Die Mehrzahl der vorgeschlagenen Katalysatoren enthalten Vanadin als einen der Bestandteile und nachdem gefunden worden war, dass Phosphor die Selektivität der Maleinsäureanhydrid-Bildung stark erhöht, auch dieses. Normalerweise liegen diese Bestandteile als Vanadylphosphat vor, in dem Vanadin ganz oder teilweise zu einer niederen Oxidationsstufe als 5 reduziert ist. In den meisten Fällen ist das Vanadylphosphat noch mit den unterschiedlichsten Stoffen aus der Reihe der Alkalien, Erdalkalien und der Übergangselemente des Periodensystems der Elemente zur Erhöhung der Aktivität und/oder der Lebensdauer der Katalysatoren promotiert. Das Vanadylphosphat ist jedoch, von Ausnahmen abgesehen, Hauptbestandteil dieser Katalysatoren. Die in einigen Fällen vorgeschlagenen V-freien Katalysatoren sind meist nur für die Oxidation von Butadien brauchbar, allenfalls noch für die Butene, nicht aber für Butan.

Die Aktivität von Vanadylphosphat-Katalysatoren ist stark von der Art ihrer Herstellung abhängig. Normalerweise findet diese in wässeriger Lösung statt in Gegenwart von HCl oder Oxalsäure als Reduktionsmittel oder auch mit $H_3PO_3$, wie Sen Gupta, Pat und Mukherjee, „J.C.S.", Dalton, 1974, 226 beschrieben haben. Diese Verfahren haben aber den Nachteil, dass grosse Mengen Flüssigkeit zur Trockne eingeengt werden müssen, um die aktive Masse – das Katalysatorvorprodukt – zu gewinnen und dass ein bestimmtes, meist langwieriges Eintrocknungsprogramm einzuhalten ist, wenn das Katalysatorvorprodukt eine genügende Aktivität entfalten soll. Es ist z.B. ein schnelles Eindampfen im Rotationsverdampfer im Vakuum oder im Sprühtrockner nicht anwendbar, da die Aktivität bei einem zu schnellen Entfernen des Wassers aus dem Kristallgitter zu stark vermindert wird.

Auf der Suche nach anderen Verfahren hat man vorgeschlagen, die Herstellung in organischen Lösungsmitteln durchzuführen, wobei meist niedere aliphatische Alkohole verwendet wurden. Es wird gemäss den deutschen Offenlegungsschriften DE-A Nrn. 2427154 und 2328755 Isobutanol und HCl-Gas und gemäss der deutschen Offenlegungsschrift Nr. 2700635, sowie den US-Patentschriften Nrn. 4062873 und 4064070 Isobutanol und Benzylalkohol sowohl als Lösungsmittel als auch als Reduktionsmittel angewendet. Der Nachteil dieser Verfahren ist der Umgang mit grossen Mengen gasförmiger HCl, die zunächst in die Aufschlämmung von $V_2O_5$ in Isobutanol eingeleitet, während des Rückflusserhitzens aber wieder frei werden und aufzufangen sind.

Ein weiterer gravierender Nachteil ist die Tatsache, dass der Katalysatorkomplex in Lösung geht, woraus das Katalysatorvorprodukt nur durch Einengen der Lösung gewonnen werden kann. Zudem wurde festgestellt, dass die Reproduzierbarkeit der Katalysatoraktivität nach dieser Methode nicht gegeben ist. Die Verwendung von Benzylalkohol hat zwar den Vorteil, dass der Katalysator im Medium schwer löslich ist, demnach ausfällt und durch Filtration gewonnen werden kann, dagegen aber den Nachteil, dass die Reduktionsgeschwindigkeit der Alkohole gering ist und infolgedessen lange Rückflusszeiten erforderlich sind. Ausserdem muss noch Wasser azeotrop entfernt werden, um genügend Aktivität zu erhalten. Unterbleibt dies, so fällt die Aktivität stark ab. Zudem führt der entstehende Benzaldehyd zu Kondensationen und Verstopfungen beim Aufarbeiten und Anfahren des Katalysators.

Es war daher Aufgabe der Erfindung, die genannten Nachteile bei der Herstellung des Vanadylphosphat-Katalysators zu vermeiden und ausserdem einen Katalysator zu entwickeln, der zu hoher Selektivität und Ausbeute im Verfahren führt und sich sowohl für die Oxidation von Butenen, von Butadien als auch von Butan bzw. deren Gemischen verwenden lässt. Dies wurde durch die Kombination verschiedener Massnahmen, wie nachstehend angezeigt, erreicht.

Gegenstand der Erfindung ist daher ein Verfahren zur Herstellung von Katalysatoren für die Gasphaseoxidation von gesättigten und/oder ungesättigten Kohlenwasserstoffen zu Maleinsäureanhydrid auf Basis Vanadylphosphat ausgehend von einer Vanadinverbindung mit fünfwertigem Vanadin und Orthophosphorsäure in einem Alkohol aus der Reihe der einwertigen niederen aliphatischen Alkohole mit 2 bis 8 C-Atomen als Suspensionsmittel und gegebenenfalls in Gegenwart von Promotoren, dadurch gekennzeichnet, dass man zur Reduktion des Vanadins die Reduktionsmittelkombination $H_3PO_3$ + Alkohol verwendet, wobei $H_3PO_3$ in solch unterstöchiometrischen Mengen eingesetzt wird, dass diese nur zur Reduktion des Vanadins auf eine Oxidationsstufe von 4,2 bis 4,4, bevorzugt 4,3, ausreicht und die weitere Reduktion des Vanadins zu einer Oxidationsstufe zwischen 3,9 und 4,0, bevorzugt 3,95 bis 3,98, mit Hilfe des Alkohols durchführt, das gegebenenfalls mit der $H_3PO_4$ eingebrachte Wasser als Azeotrop mit dem verwendeten Alkohol abdestilliert und nach Bildung des Vanadylphosphats ein durch Hochtemperaturpyrolyse von Titantetrahalogeniden gewonnenes $TiO_2$ und gegebenenfalls die

weiteren Promotoren Ni, Fe, Li, Mg zusetzt, den ausgefallenen Katalysator nach Abkühlen, Filtrieren und Trocknen in bekannter Weise verformt und die Aktivierung des Katalysators im Oxidationsreaktor selbst bei Reaktionstemperaturen von 450 bis 510° C während 12 bis 72 h in Gegenwart eines Luft/Kohlenwasserstoff-Stromes durchführt.

Es wurde gefunden, dass sich $H_3PO_3$ ebenso glatt wie in Wasser auch in primären, sekundären und tertiären Alkoholen, bevorzugt mit 2 bis 8 C-Atomen, als Reduktionsmittel verwenden lässt. Von besonderem Vorteil erwies sich der Einsatz von Isobutanol. Überraschend war, dass die Reaktionsgeschwindigkeit dabei sehr hoch ist. Da Sen Gupta, Pat und Mukherjee angeben, dass die Reaktionsgeschwindigkeit in Wasser ausser von der Temperatur auch von der Acidität der Lösung abhängig ist, konnte nicht ohne weiteres vorausgesehen werden, dass die Acidität von $H_3PO_4$ in Isobutanol genügen würde, um in kurzer Reaktionszeit zu einem vollständigen Umsatz des Reduktionsmittels $H_3PO_3$ zu kommen. Alle weiteren Möglichkeiten, den pH-Wert der Lösung zu erniedrigen, wie z.B. Zusätze von $HCl_{aq}$, Oxalsäure oder anderen organischen Säuren, haben sich als weniger brauchbare Lösungen herausgestellt. Das $PO_3^{3+}$-Ion wird bei dieser Reaktion zum $PO_4^{3+}$-Ion oxidiert. Dieses ist ein weiterer Vorteil, da das $PO_4^{3+}$-Ion ohnehin zur Bildung des Vanadylphosphats benötigt wird und auf diese Weise zur Reduktion keine fremden Stoffe einzusetzen sind. Ein weiterer Vorteil ist darin zu sehen, dass der entstehende Vanadylphosphatkomplex im verwendeten Lösungsmittel unlöslich ist und deshalb durch Filtration quantitativ gewonnen werden kann.

Während nach bekannten Verfahren, wie sie in den US-Patentschriften Nrn. 3977998, 4092269 und 4116868 und in DE-AS Nr. 2727617 beschrieben werden, $H_3PO_3$ in wässeriger Phase in Mengen angewendet wird, die mindestens der stöchiometrischen oder einer grösseren entspricht – wobei teils unter Druck im Autoklaven oder mit sehr langen Rückflusszeiten gearbeitet wird – wurde gefunden, dass es nach dem Verfahren der Erfindung günstiger ist, $H_3PO_3$ in einer kleineren Menge als der theoretischen anzuwenden und die restliche Reduktion zur gewünschten Oxidationsstufe dem Isobutanol zu überlassen. Überraschend war dabei, dass das bei dem Herstellungsprozess entstehende trockene Pulver des Katalysatorvorprodukts eine Vanadinoxidationsstufe aufwies, die in einem stets gleichbleibenden Bereich zwischen 3,90 und 4,0 vornehmlich zwischen 3,95 und 3,98 lag, unabhängig von der weiteren Behandlung des Katalysatorkomplexes nach dessen Ausfällung in Isobutanol. Das erfindungsgemässe Verfahren bezieht sich also u.a. auf eine kombinierte Reduktion des V durch $H_3PO_3$ und Alkohole, wie z.B. Isobutanol.

Nach bekannten Verfahren werden die bei der Herstellung erhaltenen Katalysatorvorprodukte meist nach der Verformung, die wiederum in bekannter Weise durch Pelletieren, Tablettieren oder Extrudieren geschehen kann, durch Calzinieren bei Temperaturen zwischen 400 und 600° C in Gegenwart oder Ausschluss von Luft in die aktive Form des Katalysators überführt. Diese Verfahrensweise hat hauptsächlich zwei Nachteile:

1. Es müssen aufwendige Apparaturen für diese Temperungen investiert werden.
2. Es treten Probleme mit der Sauerstoffempfindlichkeit des Vanadyl-IV-phosphats bei höheren Temperaturen auf, was den Ausschluss von $O_2$ oder zumindest eine beträchtliche Reduzierung oberhalb von etwa 300° C notwendig macht. Selbst das oft empfohlene Arbeiten in strömender Inertgasatmosphäre ist bei 400-600° C problematisch.

Dagegen wurde gefunden, dass es für das nach dem erfindungsgemässen Verfahren hergestellte Katalysatorvorprodukt besser ist, wenn es nach der Verformung im Reaktor selbst formiert wird. Dabei soll man bei möglichst niedrigen Temperaturen, nämlich bei einer Badtemperatur von 300-350° C, mit einem Gemisch von Luft mit einem ungesättigten und/oder gesättigten $C_4$-Kohlenwasserstoff im Gewichtsverhältnis 1:25 bis 1:50, bevorzugt 1:30 bis 1:40 anfahren, nachdem man bis zu dieser Temperatur im Luftstrom hochgeheizt hat. Die Kohlenwasserstoffbelastung beträgt dabei 10-20 g/cm² · h. Die Formierung geschieht derart, dass man die Badtemperatur so weit steigert, dass die Reaktionstemperatur *(hot spot)* Werte um 450-510° C erreicht. Die Formierung bei dieser Temperatur dauert 12-72 h. Danach senkt man die *hot spot* auf Werte um 480° C und reguliert weiter nach dem Umsatz. Die endgültige Formierung des Katalysators ist nach 10-20 d erreicht, woran man erkennt, dass eine solch behutsame und langsame Formierung kaum ausserhalb des Reaktors durchgeführt werden könnte. Will man Butan als Ausgangsmaterial fahren, so kann man so vorgehen, dass man nach dem Anfahren z.B. mit einem Butengemisch zwischen 300 und 350° C und der Steigerung der Badtemperatur auf 420-440° C bei dieser Temperatur auf Butan umschaltet und die weitere Formierung genau wie oben beschrieben durchführt. Ebenso gut wie mit Butan, Butenen bzw. Gemischen daraus kann man den Katalysator auch mit Butadien anfahren. Überraschend war, dass der nach dem Verfahren der Erfindung hergestellte und formierte Katalysator gleichermassen gut für die selektive Oxidation von ungesättigten wie auch von gesättigten $C_4$-Kohlenwasserstoffen brauchbar ist. Ebenso lässt er sich zur Oxidation von Gemischen von ungesättigten und gesättigten $C_4$-Kohlenwasserstoffen verwenden, wie sie bei der Gewinnung der $C_4$-Kohlenwasserstoffe anfallen, wie z.B. für ein Raffinat II der Zusammensetzung wie in Beispiel 1 angeführt. Bei der Oxidation solcher Gemische ist hervorzuheben, dass nach dem Verfahren der Erfindung neben dem Buten auch ein beträchtlicher Teil des anwesenden Butans umgesetzt wird, trotz der von der Oxidation reinen Butans erheblich abweichenden Reaktionsbedingungen. Im allgemeinen lassen die nach bekannten Verfahren arbeitenden Katalysatoren für die Butenoxidation das mehr oder weniger vorhandene Butan bei den entsprechenden Arbeitsbedingungen unangegriffen.

Alkohole, z.B. Isobutanol, haben vor Wasser, dessen Verwendung zur Katalysatorherstellung nach diesem Verfahren grundsätzlich auch möglich wäre, den Vorteil, dass das Vanadyl-(IV)-phosphat fast quantitativ ausfällt und so auf einfache Art und Weise durch Filtration gewonnen werden kann. Beim Arbeiten in Wasser ist das Produkt löslich und kann nur durch Eindampfen zur Trockne gewonnen werden. Von Nachteil ist auch die Tatsache, dass zur Gewinnung eines aktiven Katalysators ein bestimmtes Eindampfprogramm eingehalten werden muss. Der aus Isobutanol gewonnene Katalysator kann in beliebiger Weise mit oder ohne Vakuum getrocknet werden — ohne Nachteile für die Aktivität. Weiterhin kommt noch hinzu, dass die Reproduzierbarkeit der Katalysatoraktivität bei den in Isobutanol hergestellten Katalysatoren sehr viel besser ist als bei den in Wasser hergestellten.

Anstelle der Verwendung einer zur Erreichung einer Oxidationsstufe des Vanadins von 4,0 theoretisch erforderlichen Menge an $H_3PO_3$ ist es für die Katalysatoraktivität besser, wenn die $H_3PO_3$-Menge auf 60-80% der theoretischen Menge beschränkt wird. Besonders vorteilhaft ist es, wenn man 70% der theoretische erforderlichen Menge anwendet. Die Menge an $H_3PO_3$ reicht dabei also nur aus, die Vanadinoxidationsstufe auf 4,3 einzustellen. Da das Isobutanol selbst auch reduzierende Eigenschaften besitzt, wird die Oxidationsstufe des Vanadins im Laufe des Herstellungsprozesses weiter herabgesetzt, um schliesslich den Bereich zwischen 3,90 und 4,0 zu erreichen. Wertigkeiten zwischen 3,95-3,98 sind auf diese Weise sehr genau reproduzierbar zu erhalten. Im Hinblick auf den später durchzuführenden Aktivierungsprozess beim Anfahren des Katalysators im Reaktor ist es von grosser Wichtigkeit, dass das Katalysatorpulver bei der Herstellung im Chargenbetrieb mit einer einheitlichen Vanadinoxidationsstufe anfällt.

Grundsätzlich kann der Ansatz auch ohne Entfernen des Wassers durchgeführt werden. Das hat aber den Nachteil, dass bei der Verwendung eines hochdispersen $TiO_2$ in Anwesenheit von Wasser eine sehr dickflüssige Suspension entsteht, die — auch bei Verdünnung — schlecht filtrierbar ist und einen Katalysator mit verminderter Aktivität ergibt. Diesen Nachteil könnte man durch Verwendung von 100%iger $H_3PO_4$ umgehen, jedoch ist diese infolge ihrer physikalischen Eigenschaften sehr schlecht handhabbar. Vorzuziehen ist deshalb eine etwa 85%ige $H_3PO_4$.

Da die P-Verbindungen in Isobutanol gut löslich sind, nicht aber das $V_2O_5$, findet die Reaktion in heterogener Phase statt. Trotzdem ist bei Rückflusstemperatur die Reaktionsgeschwindigkeit der Partner so gross, dass die Gesamtreaktion in 4-8 h beendet ist. Das durch die 85%ige $H_3PO_4$ eingebrachte Wasser wird innerhalb der ersten 60 min durch Abdestillieren ausgeschleust. Wasser und Isobutanol bilden ein Azeotrop, das bei 90°C siedet. Wenn innerhalb 1 h etwa 15% des ursprünglich vorgelegten Isobutanols abdestilliert wird, ist das Wasser auch ohne wirksame Kolonne in ausreichendem Masse entfernt. Die Kopftemperatur steigt dabei von 90 auf etwa 100°C an.

$H_3PO_4$ wird so dosiert, dass zusammen mit der $H_3PO_3$ ein P/V-Atomverhältnis von 1,2 vorhanden ist. Das ist ein geringer Überschuss über die benötigte Menge, denn das fertige Vanadyl-IVphosphatpulver hat stets ein P/V-Verhältnis von 1,10. Die restliche $H_3PO_4$-Menge bleibt in der Isobutanolmutterlauge gelöst. Ein geringer Säureüberschuss ist wichtig, da die Reduktion des V durch $H_3PO_3$ und Isobutanol nur in saurer Lösung genügend schnell abläuft.

Ti wird in Mengen zugesetzt, dass das Atomverhältnis zu V zwischen 0,05 und 0,4 liegt, und zwar in Form von $TiO_2$, das z.B. durch Hochtemperaturpyrolyse von $TiCl_4$ gewonnen wird. Es besitzt eine höhere Oberfläche als die normalen Handelsformen des Anatas oder Rutils (50-80 $m^2$/g gegen 5-20 $m^2$/g) und verleiht dem Katalysator eine grössere Aktivität als z.B. normaler Anatas.

Die Promotoren Ni, Fe, Li und Mg werden nicht in Form eines ihrer handelsüblichen Salze eingesetzt. Es hat sich auf Grund der unterschiedlichen Löslichkeiten der Salze in Isobutanol als vorteilhaft erwiesen, durch Tempern von Salzgemischen bei 500-600°C in Isobutanol unlösliche oder schwer lösliche Oxidgemische herzustellen, die dann gegebenenfalls zusammen mit dem hochdispersen $TiO_2$ dem Ansatz zugesetzt werden, wenn das Wasser azeotrop entfernt ist. In vorteilhafter Weise verwendet man zum Tempern Verbindungen wie Hydroxide, Hydroxidhydrate, Hydroxicarbonate oder Nitrate. Die Promotoren werden in solchen Mengen zugesetzt, dass die Atomverhältnisse von Ni oder Fe zu V zwischen 0,01 und 0,05 und die von Li oder Mg zu V zwischen 0,001 und 0,02 liegen. Auch wenn Ni und Fe zusammen verwendet werden, sollte das Atomverhältnis der Summe zu V in dem oben für die Einzelverbindungen angegebenen Bereich liegen. Von den Elementen Li und Mg wird entweder das eine oder das andere verwendet, nicht beide gleichzeitig.

Die im experimentellen Teil angeführten Begriffe wie Umsatz, Ausbeute und Selektivität werden wie im folgenden definiert angewendet:

$$\text{Umsatz in \%} = \frac{\text{g KW umgesetzt} \cdot 100}{\text{g KW-Einsatz}} = U$$

$$\text{Ausbeute MSA in Gew.-\%} = \frac{\text{g MSA} \cdot 100}{\text{g KW-Einsatz}} = A$$

$$\text{Selektivität in Mol-\%} = \frac{\dfrac{\text{g MSA gef.} \cdot 10^4}{\text{g MSA Theorie} \cdot U}}{100} = S$$

Ausserdem ist die
Belastung in g/$cm^2 \cdot$ h = g KW pro $cm^2$ Rohrquerschnitt und Stunde

und das
Gewichtsverhältnis

$\dfrac{\text{Luft}}{\text{KW}}$ z.B. 35 = die Menge Luft in g pro 9 KW

In den Beispielen werden folgende Abkürzungen verwendet:

SBT = Salzbadtemperatur
RT = Reaktionstemperatur in der *hot spot*-Zone
MSA = Maleinsäureanhydrid
KW = Kohlenwasserstoff
U = Umsatz
A = Ausbeute
S = Selektivität

*Beispiele:*

*Beispiel 1:*

A. *Katalysatorherstellung*

In 28 l Isobutanol werden bei Raumtemperatur unter Rühren 1033 g $H_3PO_3$ (ausreichend zum Erreichen einer V-Oxidationsstufe von 4,3 bei quantiativem Umsatz) gelöst und 3528 g 85%ige $H_3PO_4$ zugegeben. In dieser Lösung werden dann 3274 g $V_2O_5$ aufgeschlämmt und das Ganze zum Sieden erhitzt. Vom Rückfluss werden innerhalb 30-60 min etwa 5 l Isobutanol abgenommen. Dieses Destillat enthält das mit der 85%igen $H_3PO_4$ eingegebene freie Wasser, das auf diese Weise aus dem Ansatz entfernt wird. Nun gibt man 576 g $TiO_2$ (Oberfläche: 56 m²/g) hinzu und hält die Suspension weitere 5 h auf Rückfluss.

Danach wird der Ansatz auf Raumtemperatur abgekühlt, das ausgefallene hellblaue Produkt abgeschleudert und 12 h bei 130°C im Vakuum bei 25-50 mbar getrocknet. Man erhält 6,8 kg eines grauen Katalysatorvorproduktes mit einer Oberfläche von 31 m²/g und einer Vanadinoxidationsstufe von 3,98. Seine Zusammensetzung ist V:P:Ti = 1:1, 10:0,20.

Dieses Katalysatorvorprodukt wird mit 3% Graphit versetzt und zu Tabletten mit einem ∅ von 6 mm und einer Dicke von 4-5 mm verpresst. Das Schüttgewicht dieser Tabletten liegt bei 750-800 g/l.

B. *Aktivieren des Katalysators*

Hierzu wird ein Reaktor verwendet mit Salzbadkühlung und einem Reaktionsrohr von 3,50 m Länge und einem Innendurchmesser von 25 mm. Das Reaktionsrohr ist versehen mit einem Thermorohr von 6 mm Aussendurchmesser. Der Reaktor wird mit 1110,17 ml des obigen Katalysators gefüllt, was einer Füllhöhe von 240 cm entspricht. Der Rohrquerschnitt beträgt unter Berücksichtigung des Thermorohres 4,6257 cm².

Der Katalysator wird mit einem Luftstrom von 500 l/h beaufschlagt und das Salzbad aufgeschmolzen und auf eine Temperatur von 200°C gebracht. Unter weiterem Luftdurchleiten wird in 26 h von 200 auf 330°C aufgeheizt. Ab 330°C leitet man ein Buten-Luftgemisch über den Katalysator in einer Menge von 15 g/cm² · h Buten und einem Luft/Buten-Gewichtsverhältnis von 35. Das verwendete technische Buten hatte folgende Zusammensetzung:

n-Butan: 20,19%
iso-Butan: 5,81%
Buten-1: 46,73%

cis-Buten-2: 10,82%
trans-Buten-2: 16,20%

Nun erhöht man die Salzbadtemperatur von 330°C langsam mit etwa 5°C/h bis die Reaktionstemperatur in der Reaktionszone *(hot spot)* 500°C erreicht hat (Badtemperatur etwa 420°C) und hält sie 12 h auf dieser Höhe. Während dieser Zeit erhöht man die Belastung auf 20 g/cm² · h unter Beibehaltung des Luft/Buten-Verhältnisses. Die Raumvolumgeschwindigkeit (RVG) beträgt nunmehr 2290 h⁻¹. Die Badtemperatur wird nach der *hot spot* einreguliert. Nach den 12 h senkt man sie so weit, dass der Umsatz 85-90% beträgt. In den nächsten 8-14 d findet eine weitere langsame Aktivierung statt, in deren Verlauf die Badtemperatur bei konstantem Umsatz weiter gesenkt werden kann. Schliesslich stellt sich eine *hot spot*-Temperatur von 465°C bei einer Badtemperatur von 380°C ein.

C. *Ergebnis der Katalysatortestung*

Der nach A hergestellte und nach B angefahrene und aktivierte Katalysator zeigt mit dem unter B angegebenen Buten als Ausgangsmaterial die in Tabelle 1 angegebenen Ergebnisse bei einer Belastung von 20 g/cm² · h einem Luft/Butan-Gewichtsverhältnis von 35 und bei einem Katalysatorvordruck von 400 mbar:

Tabelle 1

| Laufzeit Tage | SBT (°C) | RT (°C) | U (%) | A Gew.-% | S Mol-% |
|---|---|---|---|---|---|
| 40 | 380 | 465 | 87,0 | 75,0 | 49,3 |
| 100 | 380 | 465 | 87,0 | 74,0 | 48,7 |
| 200 | 382 | 460 | 86,0 | 71,5 | 47,6 |

*Beispiel 2:*

Dieses Beispiel beschreibt den Einsatz von Butan (Gehalt: 98,7%, 1% iso-Butan, 0,3% Butene) als Ausgangsmaterial an dem nach Beispiel 1 A hergestellten Katalysator.

B. *Aktivierung des Katalysators*

Der Katalysator wird in dem gleichen Reaktor und in der gleichen Weise, wie im Beispiel 1 beschrieben, mit dem technischen Buten der im Beispiel 1 angegebenen Zusammensetzung bei 330°C angefahren und die Salzbadtemperatur erhöht, bis die *hot spot* 500°C erreicht hat. Man bringt die Salzbadtemperatur eventuell unter Zurücknahme der Butenmenge ohne Änderung der Luftmenge auf 430°C. Bei dieser Temperatur wird das Buten durch Butan ersetzt, derart, dass eine Belastung von 15 g/cm² · h resultiert. Da der Luftstrom dabei nicht geändert wurde, beträgt das Luft/Butan-Gewichtsverhältnis 35. Nachdem das Buten durch Butan verdrängt ist, geht die *hot spot*-Temperatur zurück auf Werte um 15-30°C über der Badtemperatur. Man erhöht die Badtemperatur auf 450°C. Dabei steigt die *hot spot*-Temperatur langsam auf Werte um 500°C. Man reguliert die Badtemperatur so ein, dass die *hot spot*-Temperatur 24 h auf dieser Höhe bleibt. In dieser Zeit erhöht man die Belastung von 15 auf 20 g/cm² · h unter Beibehaltung des Luft-Butan-Verhältnisses. Danach senkt man die *hot spot*-Temperatur auf 480°C unter entsprechender Rücknahme der Badtemperatur. Im Laufe der nächsten 8-14 d findet eine weitere langsame Aktivierung statt, in deren Verlauf man die *hot spot*-Temperatur weiter senken kann, derart, dass der Umsatz 90% nicht übersteigt.

### C. *Ergebnis der Katalysatortestung*

Mit Einsatz des oben angegebenen Butans als Rohstoff erhält man bei einer Belastung von 20 g/cm² · h, einem Luft/Butan-Gewichtsverhältnis von 35 und einem Katalysatorvordruck von 400 mbar, die in Tabelle 2 angeführten Ergebnisse:

Tabelle 2

| Laufzeit Tage | SBT (°C) | RT (°C) | U (%) | A Gew.-% | S Mol-% |
|---|---|---|---|---|---|
| 35 | 411 | 455 | 85,0 | 82,5 | 57,5 |
| 80 | 408 | 450 | 84,0 | 80,7 | 56,9 |
| 150 | 410 | 450 | 84,3 | 79,8 | 56,1 |

*Beispiel 3:*

Ein nach Beispiel 1 A hergestellter Katalysator wird in einen Reaktor gefüllt, dessen Reaktionsrohr einen ⌀ von 22,6 mm und eine Länge von 60 cm besitzt. Er ist versehen mit einem Thermorohr mit dem äusseren ⌀ von 6 mm. Das Katalysatorvolumen beträgt 186,4 ml bei einer Füllhöhe von 50 cm. Der Katalysator wird im Luftstrom in 4 h auf eine Salzbadtemperatur von 350°C gebracht und bei dieser Temperatur mit einem Butadien-Luftgemisch mit einer Raumvolumgeschwindigkeit von 1250 h⁻¹ und einem Luft/Butadien-Gewichtsverhältnis von 40 angefahren. Dann senkt man die Salzbadtemperatur solange, bis die maximale Ausbeute erreicht ist. Das ist bei 320°C der Fall. Bei quantitativem Umsatz erhält man dabei eine Ausbeute von 112 Gew.-% bezogen auf das eingesetzte Butadien, was einer Selektivität von 61,8 Mol-% entspricht.

*Beispiel 4:*

Es wird ein Katalysator hergestellt nach dem Verfahren von Beispiel 1 A nur mit dem Unterschied, dass mit dem TiO₂ zusammen ein durch Tempern von Nickelhydroxycarbonat bei 500°C hergestelltes Nickeloxid in einer solchen Menge zugegeben wird, dass das Atomverhältnis von Ni:V = 0,02:1 beträgt. Nach der Aufarbeitung hat das Katalysatorpulver folgende Zusammensetzung: V:P:Ti:Ni = 1:1, 10:0,20:0,02.

Dieser Katalysator wird tablettiert und in den im Beispiel 1 B beschriebenen Reaktor gefüllt und in folgender Weise angefahren:

Der Reaktor wird im Luftstrom auf 350°C hochgeheizt. Ab dieser Temperatur leitet man ein Butan/Luft-Gemisch über den Katalysator in einer Menge von 15 g/cm · h Butan und einem Luft-Butan-Gewichtsverhältnis von 35. Man steigert die Salzbadtemperatur langsam mit 5°/h, bis die Reaktionstemperatur in der *hot spot*-Zone etwa 500°C erreicht hat und hält diese Reaktionstemperatur 24 h aufrecht. Dann senkt man die Salzbadtemperatur, bis die Reaktionstemperatur 480°C beträgt. Die Temperatur bleibt auf dieser Höhe solange, bis der Umsatz Werte zwischen 85 und 90% erreicht, was einige Tage in Anspruch nimmt. Danach reguliert man die Salzbadtemperatur so ein, dass der Umsatz etwa 85% beträgt. Bei einer Belastung von 20 g Butan/cm² · h, einem Luft/Butan-Gewichtsverhältnis von 35 und einem Katalysatorvordruck von 400 mbar erhält man das in Tabelle 4 angegebene Ergebnis:

Tabelle 4

| Laufzeit Tage | SBT (°C) | RT (°C) | U (%) | A Gew.-% | S Mol-% |
|---|---|---|---|---|---|
| 28 | 410 | 460 | 87,2 | 84,6 | 57,5 |
| 65 | 406 | 455 | 86,5 | 84,9 | 58,1 |
| 108 | 405 | 455 | 86,1 | 84,1 | 57,8 |

*Beispiel 5:*

Es wird ein Katalysator hergestellt nach dem Verfahren von Beispiel 1 A mit dem Unterschied, dass mit dem TiO₂ zusammen ein bei 500°C getempertes Gemisch von Nickelhydroxycarbonat und LiOH · H₂O zugegeben wird in einer solchen Menge, dass die Atomverhältnisse Ni/V = 0,03:1 und Li/V = 0,01:1 entstehen. Nach der Aufarbeitung hat der Katalysator die Zusammensetzung V/P/Ti/Ni/Li = 1:1,10:0,20:0,03:0,01. Dieser Katalysator wird tablettiert und in den im Beispiel 1 B beschriebenen Reaktor gefüllt und nach Beispiel 2 B angefahren und aktiviert.

Mit Butan als Ausgangsmaterial, einer Belastung von 20 g/cm² · h, einem Luft/Butan-Verhältnis von 35 und einem Katalysatorvordruck von 400 mbar, erhält man das in Tabelle 5 angegebene Ergebnis:

Tabelle 5

| Laufzeit Tage | SBT (°C) | RT (°C) | U (%) | A Gew.-% | S Mol-% |
|---|---|---|---|---|---|
| 30 | 415 | 465 | 82,4 | 80,4 | 57,8 |
| 60 | 412 | 460 | 81,9 | 81,2 | 58,7 |
| 95 | 410 | 455 | 79,6 | 80,7 | 60,1 |

*Beispiel 6:*

Es wird ein Katalysator hergestellt nach dem Verfahren von Beispiel 1 A nur mit dem Unterschied, dass mit dem $TiO_2$ zusammen ein durch Tempern von $Fe(NO_3)_2 \cdot 9\,H_2O$ und Magnesiumhydroxycarbonat bei 500°C hergestelltes Gemisch der beiden Oxide in einer solchen Menge zugegeben wird, dass die Atomverhältnisse Fe/V = 0,02:1 und Mg/V = 0,005:1 betragen. Nach der Aufarbeitung hat der Katalysator die Zusammensetzung V/P/Ti/Fe/Mg = 1:1,10:0,2:0,02:0,005.

Dieser Katalysator wird tablettiert und in den im Beispiel 1 B beschriebenen Reaktor eingefüllt und nach Beispiel 2 B angefahren und aktiviert.

Mit Butan als Ausgangsmaterial, einer Belastung von 20 g/cm² · h, einem Luft/Butan-Gewichtsverhältnis von 35 und einem Katalysatorvordruck von 400 mbar, erhält man das in Tabelle 6 angegebene Ergebnis:

Tabelle 6

| Laufzeit Tage | SBT (°C) | RT (°C) | U (%) | A Gew.-% | S Mol-% |
|---|---|---|---|---|---|
| 25 | 412 | 470 | 94,3 | 78,4 | 49,3 |
| 50 | 408 | 460 | 91,4 | 81,6 | 52,9 |
| 70 | 406 | 460 | 90,8 | 80,7 | 52,7 |

*Beispiel 7:*

Es wird ein Katalysator hergestellt nach dem Verfahren vom Beispiel 1 A nur mit dem Unterschied, dass mit dem $TiO_2$ zusammen ein durch Tempern von Nickelhydroxycarbonat, $Fe(No_3)_2 \cdot 9\,H_2O$ und $LiOH \cdot H_2O$ bei 500°C hergestelltes Oxidgemisch in einer solchen Menge zugegeben wird, dass die Atomverhältnisse Ni/V = 0,01:1, Fe/V = 0,02:1 und Li/V = 0,01 betragen. Nach der Aufarbeitung hat der Katalysator die Zusammensetzung V/P/Ti/Ni/Fe/Li = 1:1,10:0,20:0,02:0,02:0,01.

Dieser Katalysator wird als Tablette in den Reaktor entsprechend Beispiel 1 B eingesetzt und nach Beispiel 2 B angefahren und aktiviert.

Mit Butan als Ausgangsmaterial, einer Belastung von 20 g/cm² · h, einem Luft/Butan-Gewichtsverhältnis von 35 und einem Katalysatorvordruck von 400 mbar erhält man das in Tabelle 7 angegebene Ergebnis:

Tabelle 7

| Laufzeit Tage | SBT (°C) | RT (°C) | U (%) | A Gew.-% | S Mol-% |
|---|---|---|---|---|---|
| 21 | 408 | 465 | 88,4 | 83,9 | 56,3 |
| 48 | 401 | 460 | 87,3 | 83,0 | 56,4 |
| 85 | 398 | 455 | 85,8 | 82,9 | 57,3 |

*Beispiel 8 (Vergleichsbeispiel):*

Dieses Beispiel soll den Unterschied zeigen zwischen der Verwendung von normalem $TiO_2$ in Anatasform und der von uns beanspruchten, besonderen, hochdispersen Form.

Ein nach Beispiel 1 A hergestellter Katalysator wird in einen Reaktor eingefüllt, dessen Reaktionsrohr einen $\varnothing$ von 22,6 mm und eine Länge von 60 cm besitzt. Er ist versehen mit einem Thermorohr mit dem äusseren $\varnothing$ von 6 mm. Das Katalysatorvolumen beträgt 186,4 ml bei einer Füllhöhe von 50 cm. Der Katalysator wird in 4 h im Luftstrom auf 350°C gebracht, bei 350°C mit einem Butan/Luft-Strom angefahren mit einer Raumvolumgeschwindigkeit von f−1 und einem Luft/Butan-Gewichtsverhältnis von 30. Die Salzbadtemperatur wird in etwa 3 h von 350°C an soweit angehoben, dass die Reaktionstemperatur *(hot spot)* 500°C erreicht; nachdem diese Temperatur einige Stunden gehalten worden ist, senkt man die Salzbadtemperatur soweit, bis die maximale MSA-Ausbeute erreicht ist.

Dasselbe Verfahren führt man durch mit einem nach Beispiel 1 A hergestellten Katalysator, bei dem man das $TiO_2$ mit einer Oberfläche von 56 m²/g durch Anatas (O = 10,5 m²/g) ersetzt hat.

Tabelle 8 zeigt die mit beiden Katalysatoren erhaltenen Ergebnisse:

Tabelle 8

| Katalysator | SBT (°C) | U (%) | Amax Gew.-% | S Mol-% |
|---|---|---|---|---|
| Original Vergleichs-katalysator | 390 | 88,5 | 88,5 | 59,3 |
| mit Anatas | 390 | 84,0 | 78,5 | 55,4 |

Hieraus geht klar hervor, dass die unter diesen Bedingungen erreichbare maximale Ausbeute bei Verwendung des hochdispersen $TiO_2$ wesentlich höher liegt als bei Verwendung von $TiO_2$ in Anatasform. Auch andere handelsübliche Formen des $TiO_2$, gleichgültig, ob in Anatas- oder Rutilform, fallen gegenüber dem beanspruchten hochdispersen $TiO_2$ deutlich ab.

*Beispiel 9 (Vergleichsbeispiel):*

In diesem Beispiel wird der nach Beispiel 1 A hergestellte Katalysator mit zwei anderen nach der gleichen Methode hergestellten Katalysatoren verglichen, bei denen aber mehr Reduktionsmittel $H_3PO_3$ angewendet worden ist.

Der Katalysator nach Beispiel 1 A enthält soviel Reduktionsmittel $H_3PO_3$, dass sich bei alleiniger Reduktion durch $H_3PO_3$ eine Vanadinoxidationsstufe $V^x$ von 4,3 einstellen würde. Nach der Methode von Beispiel 1 A wurden zwei weitere Katalysatoren hergestellt, bei denen die $H_3PO_3$-Menge derart erhöht wurde — unter entsprechender Verringerung der Menge an $H_3PO_4$, damit das

P/V-Verhältnis konstant bleibt — dass sich eine Vanadinoxidationsstufe $V^x$ von 4,1 bzw. 4,0 ergeben würde. Die 3 Katalysatoren wurden in dem im Vergleichsbeispiel 8 angegebenen Reaktor mit der gleichen Anfahrmethode mit Butan getestet und ihre maximalen Ausbeuten an MSA ermittelt. Dabei erhält man das in Tabelle 9 wiedergegebene Ergebnis:

Tabelle 9

| Katalysator | SBT (°C) | U (%) | Amax Gew.-% | S Mol-% |
|---|---|---|---|---|
| $V^x$ = 4,3 | 390 | 88,5 | 88,5 | 59,3 |
| $V^x$ = 4,1 | 440 | 87,3 | 75,1 | 51,0 |
| $V^x$ = 4,0 | 440 | 83,6 | 71,9 | 51,0 |

Die Erhöhung der Menge des Reduktionsmittels $H_3PO_3$ führt also zur Verringerung der Katalysatoraktivität; die maximale Ausbeute verringert sich und wird ausserdem erst bei einer wesentlich höheren Badtemperatur erreicht.

*Beispiel 10 (Vergleichsbeispiel):*

In diesem Beispiel wird ein nach der Methode des Beispiels 1 A hergestellter Katalysator (1 A) mit einem solchen verglichen, bei dem während der Herstellung das azeotrope Entfernen des Wassers unterbleibt (1 B).

Die beiden Katalysatoren werden in dem im Vergleichsbeispiel 8 beschriebenen Reaktor mit der gleichen Anfahrprozedur mit einem Butan/Luft-Gemisch wie dort getestet und die maximale Ausbeute an MSA innerhalb der ersten 4 d ermittelt. Das Ergebnis zeigt Tabelle 10.

Tabelle 10

| Katalysator | SBT (°C) | U (%) | Amax Gew.-% | S Mol-% |
|---|---|---|---|---|
| 1 A | 390 | 88,5 | 88,5 | 59,3 |
| 1 B | 400 | 86,3 | 78,9 | 54,2 |

Man sieht, dass die grössere Menge Wasser, die im Katalysator 1 B während der Herstellung anwesend ist, die Anfangsaktivität des Katalysators dämpft, erkenntlich an der niedrigen maximalen Ausbeute und der höheren Badtemperatur, bei der diese erreicht wird.

**Patentansprüche**

1. Verfahren zur Herstellung von Katalysatoren für die Gasphaseoxidation von gesättigten und/oder ungesättigten $C_4$-Kohlenwasserstoffen zu Maleinsäureanhydrid auf Basis Vanadylphosphat ausgehend von einer Vanadinverbindung mit fünfwertigem Vanadin und Orthophosphorsäure in einem Alkohol aus der Reihe der einwertigen niederen aliphatischen Alkohole mit 2 bis 8 C-Atomen als Suspensionsmittel und gegebenenfalls in Gegenwart von Promotoren, dadurch gekennzeichnet, dass man zur Reduktion des Vanadins die Reduktionsmittelkombination $H_3PO_3$ + Alkohol verwendet, wobei $H_3PO_3$ in solch unterstöchiometrischen Mengen eingesetzt wird, dass diese nur zur Reduktion des Vanadins auf eine Oxidationsstufe von 4,2 bis 4,4, bevorzugt 4,3, ausreicht und die weitere Reduktion des Vanadins zu einer Oxidationsstufe zwischen 3,9 und 4,0, bevorzugt 3,95 bis 3,98, mit Hilfe des Alkohols durchführt, das gegebenenfalls mit der $H_3PO_4$ eingebrachte Wasser als Azeotrop mit dem verwendeten Alkohol abdestilliert und nach Bildung des Vanadylphosphats ein durch Hochtemperaturpyrolyse von Titantetrahalogeniden gewonnenes $TiO_2$ und gegebenenfalls die weiteren Promotoren Ni, Fe, Li, Mg zusetzt, den ausgefallenen Katalysator nach Abkühlen, Filtrieren und Trocknen in bekannter Weise verformt und die Aktivierung des Katalysators im Oxidationsreaktor selbst bei Reaktionstemperaturen von 450 bis 510°C während 12 bis 72 h in Gegenwart eines Luft/Kohlenwasserstoffstromes durchführt.

2. Verfahren nach Anspruch 1, dadurch gekennzeichnet, dass Ti als alleiniger Promotor in Form eines durch Hochtemperaturpyrolyse von Titantetrahalogeniden, insbesondere von $TiCl_4$, gewonnenen $TiO_2$ mit einer Oberfläche zwischen 50 und 80 m²/g in einer Menge, dass das Atomverhältnis Ti zu V zwischen 0,05 und 0,4, bevorzugt zwischen 0,15 und 0,25 liegt, eingesetzt wird.

3. Verfahren nach Anspruch 1, dadurch gekennzeichnet, dass als weitere Promotoren Nickel und Eisen einzeln oder beide zusammen, gegebenenfalls in Verbindung mit entweder Lithium oder Magnesium, in Form von durch Tempern von Salzen dieser Elemente bei 500-600°C gewonnenen Oxiden verwendet werden.

4. Verfahren nach Anspruch 1, dadurch gekennzeichnet, dass die Atomverhältnisse Ni oder Fe zu Vanadin oder Ni + Fe zu Vanadin zwischen 0,01 und 0,05 und die von Li oder Mg zu Vanadin zwischen 0,001 und 0,02 liegen.

5. Verfahren nach Anspruch 1, dadurch gekennzeichnet, dass die Summe $H_3PO_3$ und $H_3PO_4$ so bemessen wird, dass ein Atomverhältnis P zu V von 1,0 bis 1,2, bevorzugt 1,1, erreicht wird.

6. Verfahren nach Anspruch 1, dadurch gekennzeichnet, dass man den Katalysator vor der Aktivierung in einem Luftstrom auf Temperaturen zwischen 300 und 350°C aufheizt und die Luft in diesem Temperaturbereich durch ein Gemisch von Luft und einem ungesättigten und/oder gesättigten $C_4$-Kohlenwasserstoff ersetzt und die Aktivierungstemperatur von 450-510°C einstellt.

7. Verfahren nach Anspruch 1, dadurch gekennzeichnet, dass man die Aktivierung des Katalysators bei einer Kohlenwasserstoffbelastung von 10-20 g Kohlenwasserstoff/cm² · h durchführt.

8. Verfahren nach Anspruch 1, dadurch gekennzeichnet, dass man die Aktivierung des Katalysators mit einem Luft/Kohlenwasserstoff-Gemisch durchführt, bei dem das Gewichtsverhältnis Luft zu Kohlenwasserstoff 25-50:1, bevorzugt 30-40:1, beträgt.

**Revendications**

1. Procédé de préparation de catalyseurs à base de phosphate de vanadyle pour l'oxydation, en phase gazeuse, d'hydrocarbures en $C_4$ saturés et/ou insaturés en anhydride d'acide maléique à partir d'un composé de vanadium comportant du vanadium pentavalent et d'acide orthophosphorique dans un alcool choisi parmi la série des alcools aliphatiques inférieurs monovalents contenant 2 à 8 atomes de carbone, comme agent de mise en suspension, et éventuellement en présence de promoteurs, caractérisé en ce que, pour la réduction du vanadium, on utilise la combinaison d'agents réducteurs $H_3PO_3$ + alcool, $H_3PO_3$ étant utilisé en quantités hypostœchiométriques calculées de telle sorte qu'il ne soit suffisant que pour la réduction du vanadium à un degré d'oxydation de 4,2 à 4,4, de préférence de 4,3, tandis que l'on effectue la réduction complémentaire du vanadium à un degré d'oxydation compris entre 3,9 et 4, de préférence entre 3,95 et 3,98, à l'aide de l'alcool puis, par distillation, on sépare, sous la forme d'un azéotrope avec l'alcool utilisé, l'eau éventuellement introduite avec le $H_3PO_4$ et, après formation du phosphate de vanadyle, on ajoute un $TiO_2$ obtenu par pyrolyse de tétrahalogénures de titane à température élevée et l'on ajoute éventuellement les autres promoteurs, à savoir Ni, Fe, Li et Mg, on façonne le catalyseur précipité de façon connue après refroidissement, filtration et séchage et l'on effectue l'activation du catalyseur dans le réacteur d'oxydation lui-même à des températures réactionnelles de 450 à 510°C pendant 12 à 72 h en présence d'un courant d'air et d'hydrocarbure.

2. Procédé suivant la revendication 1, caractérisé en ce qu'on utilise Ti comme unique promoteur sous la forme d'un $TiO_2$ obtenu par pyrolyse de tétrahalogénures de titane (en particulier de $TiCl_4$) à température élevée et ayant une surface spécifique comprise entre 50 et 80 m²/g en une quantité calculée de telle sorte que le rapport atomique Ti/V se situe entre 0,05 et 0,4, de préférence entre 0,15 et 0,25.

3. Procédé suivant la revendication 1, caractérisé en ce que, comme promoteurs supplémentaires, on utilise le nickel et le fer individuellement ou ensemble, éventuellement en combinaison avec du lithium ou du magnésium, sous forme d'oxydes obtenus par traitement thermique des sels de ces éléments à 500-600°C.

4. Procédé suivant la revendication 1, caractérisé en ce que les rapports atomiques Ni ou Fe/V ou Ni + Fe/V se situent entre 0,01 et 0,05, tandis que le rapport atomique Li ou Mg/V se situe entre 0,001 et 0,02.

5. Procédé suivant la revendication 1, caractérisé en ce que la somme de $H_3PO_3$ et de $H_3PO_4$ est calculée de façon à atteindre un rapport atomique P/V de 1 à 1,2, de préférence de 1,1.

6. Procédé suivant la revendication 1, caractérisé en ce que, avant l'activation, on chauffe le catalyseur dans un courant d'air à des températures comprises entre 300 et 350°C et, dans cet intervalle de températures, on remplace l'air par un mélange d'air et d'un hydrocarbure en $C_4$ insaturé et/ou saturé, tandis qu'on règle la température d'activation à 450-510°C.

7. Procédé suivant la revendication 1, caractérisé en ce qu'on effectue l'activation du catalyseur à une charge d'hydrocarbure de 10 à 20 g d'hydrocarbure/cm² · h.

8. Procédé suivant la revendication 1, caractérisé en ce qu'on effectue l'activation du catalyseur avec un mélange d'air/hydrocarbure dans lequel le rapport pondéral entre l'air et l'hydrocarbure est de 25 à 50:1, de préférence de 30 à 40:1.

**Claims**

1. Process for the preparation of catalysts, based on vanadyl phosphate, for the gas-phase oxidation of saturated and/or unsaturated $C_4$-hydrocarbons to maleic anhydride, which process starts from a vanadium compound containing pentavalent vanadium and orthophosphoric acid in an alcohol from the series of the monohydric lower aliphatic alcohols with 2 to 8 carbon atoms as the suspending medium, optionally in the presence of promoters, characterised in that, in order to reduce the vanadium, the reducing agent combination $H_3PO_3$ + alcohol is used, $H_3PO_3$ being employed in such sub-stoichiometric amounts that these only suffice to reduce the vanadium to an oxidation level of 4.2 to 4.4, preferably 4.2, whilst the further reduction of the vanadium to an oxidation level of 3.9 to 4.0, preferably 3.95 to 3.98, is carried out with the aid of the alcohol, that the water which may have been introduced with $H_3PO_4$ is distilled off as an azeotrope with the alcohol employed and, after formation of the ganadyl phosphate, a $TiO_2$ obtained by high-temperature pyrolysis of titanium tetrahalides is added, optionally with Ni, Fe, Li or Mg as further promoters, that the catalyst which has precipitated is moulded in a conventional manner after having been cooled, filtered off and dried, and that the activation of the catalyst is carried out in the oxidation reactor itself at reaction temperatures of 450 to 510°C for 12 to 72 h in the presence of a stream of air and hydrocarbon.

2. Process according to claim 1, characterised in that Ti is employed as the sole promoter in the form of a $TiO_2$ obtained by high-temperature pyrolysis of titanium tetrahalides, especially of $TiCl_4$, and having a surface area of between 50 and 80 m²/g, the amount used being such that the atomic ratio Ti/V is between 0.05 and 0.4, preferably between 0.15 and 0.25.

3. Process according to claim 1, characterised in that nickel and iron individually or together are used as additional promoters, optionally in conjunction with either lithium or magnesium, in the form of oxides obtained by heating salts of these elements at 500 to 600°C.

4. Process according to claim 1, characterised in that the atomic ratios of Ni or Fe/V or Ni + Fe/V are between 0.01 and 0.05, and the atomic ratios of Li or Mg/V are between 0.001 and 0.02.

5. Process according to claim 1, characterised in that the sum of $H_3PO_3$ and $H_3PO_4$ is so chosen that an atomic ratio of P/V of 1.0 to 1.2, preferably 1.1, is attained.

6. Process according to claim 1, characterised in that the catalyst is heated, before activation, in a stream of air to temperatures of between 300 and 350°C, and the air is replaced, in this temperature range, by a mixture of air and an unsaturated and/or saturated $C_4$- hydrocarbon, and the system is brought to the activation temperature of 450 to 510°C.

7. Process according to claim 1, characterised in that the activation of the catalyst is carried out at a hydrocarbon throughput of 10 to 20 g of hydrocarbon/cm² · h.

8. Process according to claim 1, characterised in that the activation of the catalyst is carried out with an air/hydrocarbon mixture in which the weight ratio of air to hydrocarbon is 25 to 50/1, preferably 30 to 40/1.